# EUROPEAN PATENT APPLICATION

(11) **EP 0 791 298 A1**
(43) Date of publication of application: **27.08.1997**
(21) Application number: 97102848.5
(22) Date of filing: 21.02.1997
(51) Int. Cl.: A23K 1/17, A23K 1/18

(54) **Growth promotion in chickens**

(30) Priority: 26.02.1996 US 606612
(71) Applicant: F. HOFFMANN-LA ROCHE AG, 4070 Basel (CH)
(72) Inventor: Untawale, Govind Gajanan, Wayne, New Jersey 07470 (US)
(74) Representative: Kellenberger, Marcus, Dr.

(57) **Abstract**

Methods and feed compositions for increasing the growth of and feed utilization in healthy chickens involving the use of frenolicin B as the active ingredient, and premixes containing said active ingredient for formulating the chicken feed compositions.

## Description

The present invention relates to the promotion of growth and feed utilization in healthy chickens and, more particularly, to a method of increasing growth and feed utilization in healthy chickens by using frenolicin B and/or a physiologically acceptable salt or ester thereof as the active ingredient(s), to chicken feed compositions and chicken feed premix compositions containing frenolicin B and/or a physiologically acceptable salt or ester thereof as the active ingredient(s), and to a method of preparing such chicken feed compositions.

Frenolicin B is a known fermentation product having antibiotic properties, as is described in e.g. U.S. Patent No. 4,199,514. Frenolicin B also has anticoccidial activity and enhances the activity of ionophorous polyether anticoccidial agents, for example lasalocid, against avian coccidiosis (see U.S. Patent No. 4,839,382).

It has now been found that the growth of and feed utilization in healthy chickens is improved, i.e. increased, by orally administering to the chickens, conveniently in combination with their feed, a sufficient amount of frenolicin B and/or a physiologically acceptable salt or ester thereof, to achieve the desired effect.

Accordingly, the present invention provides, as one aspect, a method of increasing the growth of and feed utilization in healthy chickens comprising orally administering to such chickens an effective amount of frenolicin B and/or a physiologically acceptable salt or ester thereof. The method is most conveniently effected by administering the frenolicin B to the chickens with their feed, whereby the feed contains an effective amount of frenolicin B.

For the present invention it is generally desirable to administer a sufficient amount of the frenolicin B, preferably with the chicken feed, to provide about 42 to 54 mg of frenolicin B per kilogram of body weight per day in the case of starter chickens, about 34 to 40 mg of frenolicin B per kilogram of body weight per day in the case of grower chickens, and about 33 to 35 mg of frenolicin B per kilogram of body weight per day in the case of finisher chickens. In each case the given amount of frenolicin B relates to frenolicin B itself or as active principle of a salt or ester thereof used, as appropriate. The averages of these range represent the amounts of medicament ingested with feed by the animals when the feed contains about 60 ppm of frenolicin B. These amounts can, of course, be varied depending on the size and condition of the animals.

As a further aspect the present invention provides the chicken feed composition ("medicated chicken feed") for use in the method, i e a chicken feed composition for increasing the growth of and feed utilization in chickens, in that it contains an effective amount of frenolicin B and/or a physiologically acceptable salt or ester thereof.

These compositions can be prepared by mixing the active ingredient, frenolicin B (as such or as a salt or ester thereof), either directly, or as part of a premix or concentrate, into a conventional chicken feed. It has been found that if frenolicin B is present in the feed from about 15 ppm to about 80 ppm, the desired growth promotion and feed utilization (efficiency) occurs. The preferred range is from about 30 ppm to about 60 ppm of frenolicin B.

In particular, the medicated chicken feeds can be suitably formulated by ( 1 ) adding the frenolicin B directly to conventional chicken feed and mixing, for example in a vertical feed mixer; or (2) forming a premix (also designated as concentrate) of frenolicin B by mixing it with a suitable non-toxic edible carrier, e.g. corn meal, corn distillers dried grains, soybean meal, soya grits, soya flour, wheat middlings, farina, rice grits, malt sprouts, limestone and the like and including various oils and the like for example, soya oil, which can be used in forming animal feed premixes, and then adding the premix to the feed and mixing, suitably in a feed mixer or blender.

The above indicated premix containing frenolicin B, e.g. formulated in accordance with the first step of method (2) hereinbefore, is also embraced by the present invention. This premix, intended for the formulation of a chicken feed composition as defined above, is characterized in that it contains as an active ingredient frenolicin B and/or a physiologically acceptable salt or ester thereof, the frenolicin B being present in a concentration relative to the whole premix of about 5% to about 50% by weight. The amount of the frenolicin B is conveniently selected giving consideration to the above indicated relative amounts (ma per kilogram of body weight per day) of frenolicin B administered in the method of the present invention appropriate to the age of the chickens to be fed (starter, grower or finisher). Accordingly, a aufficient amount of the frenolicin B is suitably present so that the addition of approximately one kilogram of premix to the amount of feed required to total 2,000 kilograms will provide the desired dose level.

The following Examples illustrate the invention. s

### Example 1

A floor-pen growth study utilizing ninety-eight 10 ft x 5 ft pens with built-up pine shavings litter (thin layer of fresh shavings added prior to start of study) was initiated by placing 2,450 one day-old male broiler type chicks (25 male Peterson x Arbor Acres broiler chicks per pen with average body weight range of approximately 35-45 g). Each of the 98 pens was partitioned to create a pen of 5 ft x 3.75 ft size, providing floor space of 0.75 sq. ft/bird. Upon arrival from the hatchery, boxes of 1 00 sex-separated day-old male chicks were taken in no specific order to provide the intended 25 male chicks to successive pens. As one box was emptied, another was taken until all pens contained the 25 male chicks.

Pens of 25 chicks were randomly assigned to seven dietary treatments within 14 blocks of seven adjacent pens. The seven dietary feed treatments (Table 1 ) were prepared by mixing the appropriate active ingredients with the starter, grower or finisher basal diet (Table 2) in a ribbon mixer appropriate for the batch size needed. Starter diets were crumbled, grower and finisher diets were pelleted. Birds on each treatment received the respective diet types for the following periods:

| Diet | Test Period (Days) |
|---|---|
| Starter | 1 - 21 |
| Grower | 22 - 38 |
| Finisher | 39 - 42 |

Water and feed were supplied ad libitum.

Supplemental heat was provided from brooder heat lamps in each pen for the first 3 weeks. Ventilation of the floor-pen building was controlled by manually operated exhaust fans, and thermostatically controlled heating was available as needed. No vaccinations or therapeutic treatments were administered during the course of the study.

Birds were checked (observed) at least once daily for any abnormal findings. Any chicks that died or were culled during the first five days of the test period were replaced with birds of the same sex and from the same hatch that were kept in an extra pen on a lasalocid 75 ppm diet for that purpose. After the initial five days, the weight, sex and day of removal of any bird that died or was culled were recorded and all birds that died were necropsied.

Since the birds were reared on dirt floors with reused litter as bedding, there was the possibility of the birds being infected with coccidial oocysts which are usually found in re-used litter in broiler houses. Therefore, lasalocid (at a recommended level of 75 ppm), an approved anticoccidial, was added to the feed of all birds in the study to eliminate the possibility of coccidial infection.

**Table 1**

| TRT NO. | FRENOLICIN B (PPM) | NO. REPLICATES | MALE BIRDS/PEN | TOTAL BIRDS/TREATMENT |
|---|---|---|---|---|
| 1 | 0 | 14 | 25 | 350 |
| 2 | 15 | 14 | 25 | 350 |
| 3 | 30 | 14 | 25 | 350 |
| 4 | 45 | 14 | 25 | 350 |
| 5 | 60 | 14 | 25 | 350 |
| 6 | 75 | 14 | 25 | 350 |
| 7* | VIRGINIAMYCIN | 14 | 25 | 350 |

| | | | | |
|---|---|---|---|---|
| * included as positive control. | | | | |

**Table 2**

| Ingredient Name | Percent of Feed | | |
|---|---|---|---|
| | Starter | Grower | Finisher |
| Ground yellow corn | 59.72 | 66.12 | 70.975 |
| Soybean meal | 31.20 | 24.99 | 20.296 |
| Poultry by-products | 4.00 | 4.00 | 4.000 |
| Fat, Poultry | 2.34 | 2.38 | 2.043 |
| Phosphate, defluorinated | 1.43 | 1.03 | 1.083 |
| Limestone | 0.53 | 0.71 | 0.909 |
| Sodium chloride | 0.31 | 0.31 | 0.378 |
| Vitamin premix-broiler* | 0.25 | 0.25 | 0.250 |
| Alimet (methionine liquid) | 0.17 | 0.16 | 0.170 |
| Trace minerals premix-broiler** | 0.05 | 0.05 | 0.050 |

| | | | |
|---|---|---|---|
| * Vitamin premix-broiler provides (per kg/diet): vitamin (vit.) A, 4400 IU; vit. D₃, 880 ICU; vit. E, 11 IU; riboflavin, 4.4 mg; Ca pantothenate, 9.6 mg; nicotinic acid, 44 mg; choline C1, 220 mg; vit. B12, 6.6 mcg; vit. B, 2.2 mg; menadione sodium bisulfite, 3.49 mg; folic acid, 55 mg; d-biotin, 0.11 mg; thiamine mononitrate, 2.2 mg; ethoxyquin, 125 mg. | | | |
| ** Trace minerals premix-broiler provides (in ppm of diet): Mn, 60; Zn, 50; Fe, 30; Cu, 5; I, 1.05; Ca, 75 to 90. | | | |

The parameters measured during the study were:
1. Pen weights on day O and 42 (number and total weight of males determined for each pen on day 42).
2. Feed weigh-back at day 21, 38 and 42.
3. Weight and sex of live or dead birds at the time of removal from the pen. Weights of dead and culled birds at removal were added to total live weight of pen to adjust the feed conversion.
4. Average bird weight, average weight gain, feed consumption and feed conversion calculated per pen and treatment.
5. Necropsy of dead birds.
6. Observations of daily health status.

The results of the study are presented in Table 3 below wherein the average (av.) body weight, adjusted feed/gain and mortality figures are those measured or determined at day 42:

**Table 3**

| TREATMENT | | AV. BODY WT. (grams) | ADJUSTED FEED/GAIN | MORTALITY % |
|---|---|---|---|---|
| No. | Fr.B (PPM) | | | |
| 1 | 0 | 1720 | 1.65 | 2.57 |
| 2 | 15 | 1713 | 1.63a | 2.29 |
| 3 | 30 | 1744a | 1.63a | 2.86 |
| 4 | 45 | 1739 | 1.63a | 2.29 |
| 5 | 60 | 1759a,b | 1.63a | 1.71 |
| 6 | 75 | 1741 | 1.62a | 2.00 |
| 7 | Virginiamycin 16.5 PPM | 1726 | 1.63a | 2.29 |
| Fr.B = frenolicin B | | | | |

| | | | | |
|---|---|---|---|---|
| ^{a}Significantly (P<0.05) better than treatment 1 (0 ppm Fr.B) | | | | |
| ^{b}Significantly (P<0.05) better than treatment 7 (virginiamycin) ("P" reflects the probability that the results can be reproduced: the smaller the P value, the greater the probability that the results can be reproduced) | | | | |

These results indicate that supplementation of broiler diets with frenolicin B at 30 and 60 ppm increased the average body weight over that achieved with no frenolicin B (treatment 1 ) (P<0.05). The improvement in body weight obtained with 60 ppm of frenolicin B was also significantly (P<0.05) better than with virginiamycin ( 16.5 ppm).

In the presence of 75 ppm of lasalocid as a control for coccidiosis, dietary levels from 15 to 75 ppm of frenolicin B significantly (P<0.05) improved feed conversion over that achieved with frenolicin B at 0 ppm.

No significant (P<0.05) differences in mortality were observed among the various treatment groups.

Weight and feed conversion were analyzed using standard least squares analysis of variance (ANOVA) methods (see "Basic Statistics: A Modern Approach" by Morris Hamburg, Harcourt Brace Jovanovich, Inc. 1974, e.g. pages 168-170 for tailed (t) tests). Two-way models containing terms for treatment and block main effects were fit to the weight and feed conversion data. Linear-plateau models were fit to each of the response variables. A one-sided paired tailed (t) test was used to compare treatment groups with controls. Variance was estimated by two-way ANOVA mean standard error (MSE). Linear-plateau modeling of weight indicates no improvement up to a threshold of 15 ppm, after which there is rapid improvement up to 30 ppm, with a plateau above 30 ppm. The minimum effective dose is 30 ppm for weight gain. For feed conversion there is rapid improvement from 0 to 15 ppm, with a plateau above 15 ppm. The minimum effective dose for feed conversion is 15 ppm.

### Example 2

Frenolicin B was also tested in coccidiosis-infected and non-infected broilers to show the growth promotion potential of frenolicin-B.

Studies were conducted in the following. general manner:

One day old commercial type broiler chicks were obtained from commercial hatchery and kept in wire-floored, electrically heated, battery brooders. Male birds, selected according to weight, were used. The chicks were fed experimental feeds two days before infection and maintained on the medicated feed until termination of the trial. The length of the trial ran from 1 to 29 days, 1 to 14 days or 14 to 23 days. There were six replicates of six birds each per treatment.

Eimeria field isolates from E. acervulina, E. maxima, and E. tennella were recovered from commercial operations. To evaluate the efficacy of frenolicin-B, 8.0 x 10⁵ total sporulated oocyts of the field isolates were administered per bird. The sporulated oocyts, properly volumes of 1.0 ml directly to each chick in the crop by means of a blunt needle attached to a calibrated syringe. No other drugs, for example lasalocid, were administered to the chicks.

During the study, bird group weight, feed intake and feed conversion data were recorded.

The results of this study are found in Table 4 below.

**TABLE 4**

| STUDY NO. | DRUG TREATMENT (PPM) | COCCIDIOSIS INFECTION | AVERAGE BODY WEIGHT GAIN % | AVERAGE FEED EFFICIENCY | LENGTH OF STUDY (DAYS OF AGE) |
|---|---|---|---|---|---|
| 1 | None | No | 100 | 1.83 | 1-29 |
| | Frenolicin-B (80) | No | 107 | 1.73 | |
| 2 | None | No | 100 | 1.56 | 1-14 |
| | Frenolicin-B (50) | No | 104 | 1.29 | |
| 3 | None | No | 100 | 1.75 | 14-23 |
| | Frenolicin-B (80) | No | 111 | 2.0 | |
| | None | Yes | 62 | 2.49 | 14-23 |
| | Frenolicin-B (80) | Yes | 115 | 2.33 | |
| 4 | None | No | 100 | 1.60 | 14-23 |
| | Frenolicin-B (80) | No | 99 | 1.77 | |
| | None | Yes | 47 | 3.22 | 14-23 |
| | Frenolicin-B 80 | Yes | 96 | 3.53 | |

The data show that frenolicin-B is an effective growth promoter and, as compared to infected chickens also given frenolicin-B, shows much more growth promotion activity in healthy chickens.

### Example 3

Frenolicin B (332.9 g, average assay 90.85% frenolicin B) is mixed with 5,746.54 g of rice hulls in a mixer until a homogeneous blend has been produced to form a premix.

1 kilogram of said premix can be blended with 1999 kilograms of conventional chicken feed to afford 2000 kilograms of medicated chicken feed.

## Claims

1. A method of increasing the growth of and feed utilization in healthy chickens characterized in orally administering to said chickens an effective amount of frenolicin B and/or a physiologically acceptable salt or ester thereof.

2. A method according to claim 1, wherein the method is effected by administering the frenolicin B to the chickens with their feed, whereby the feed contains an effective amount of frenolicin B.

3. A method according to claim 1 or 2, wherein the amount of frenolicin B administered in the feed is from about 15 ppm to about 80 ppm, preferably from about 30 ppm to about 60 ppm.

4. A method according to any one of claim 1 to 3, wherein the amount of frenolicin B administered per kilogram of body weight per day is about 42 to 54 mg in the case of starter chickens, about 34 to 40 mg, respectively, in the case of grower chickens, and about 33 to 35 mg, respectively, in the case of finisher chickens.

5. A chicken feed which is useful for increasing the growth of and feed utilization in healthy chickens, characterized in that it has dispersed therein, as active ingredient, an effective amount of frenolicin B and/or a physiologically acceptable salt or ester thereof.

6. A chicken feed according to claim 5, wherein the amount of active ingredient is from about 15 ppm to about 80 ppm, preferably from about 30 ppm to about 60 ppm, of frenolicin B.

7. A chicken feed composition for increasing the growth of and feed utilization in healthy chickens, characterized in that it contains as active ingredient an effective amount of frenolicin B and/or a physiologically acceptable salt or ester thereof.

8. A chicken feed composition according to claim 7, wherein the amount of active ingredient is from about 15 ppm to about 80 ppm, preferably from about 30 ppm to about 60 ppm, of frenolicin B.

9. A premix intended for the formulation of a chicken feed composition as defined in claim 7 or 8, characterized in that it contains as active ingredient frenolicin B and/or a physiologically acceptable salt or ester thereof, frenolicin B being present in a concentration of about 5% to about 50% by weight.

10. A method of formulating a chicken feed composition as defined in claim 7 or 8, characterized by (1) adding the frenolicin B directly to conventional chicken feed and mixing; or (2) forming a premix of frenolicin B by mixing it with a suitable non-toxic edible carrier and then adding the premix to the feed and mixing.
